# EUROPÄISCHE PATENTANMELDUNG

(11) **EP 1 205 211 A1**
(43) Veröffentlichungstag der Anmeldung: **15.05.2002**
(21) Anmeldenummer: 01125116.2
(22) Anmeldetag: 23.10.2001
(51) Int. Cl.: A61M 39/02, A61M 39/04

(54) **Einnadel-Zugang**

(30) Priorität: 08.11.2000 DE 10055347
(71) Anmelder: Adeva Medical Gesellschaft für Entwicklung und Vertrieb von Medizinischen Implantat-Artikeln mbH, 23556 Lübeck (DE)
(72) Erfinder: Grundei, Hans, Dr., 23558 Lübeck (DE)
(74) Vertreter: Fuchs Mehler Weiss & Fritzsche

(57) **Zusammenfassung**

Es wird ein Single-Needle-Port beschrieben, der subkutan implantierbar ist, bei dem die Portöffnung nach Beendigung der Behandlung mittels eines Verschlußelementes (4) abdichtbar ist und durch Einführung einer Punktionskanüle zur erneuten Behandlung zu öffnen ist.

Der Single-Needle-Port besteht aus einem im wesentlichen trichterförmigen Portgehäuse (1), dessen untere Trichteröffnung (2) als Portöffnung (3) ausgebildet ist und in dem das Verschlußelement (4) angeordnet ist, welches pfropfenförmig ausgebildet ist und mit seiner äußeren Mantelfläche in dichtender Anlage an dem Innenmantel des Portgehäuses (1) anliegt.

Das Verschlußelement (4) besteht aus einem homogenen, elastischen Material, durch welches hindurch die Punktionskanüle rührbar ist, wobei dann das Material die Punktionskanüle in abdichtender Weise umschließt, und welches beim Herausziehen der Portkanüle solch hohe Rückstellkräfte entwickelt, daß sich der von der Punktionskanüle ausgebildete Durchgangskanal wieder schließt. In konkreter Ausgestaltung besteht das Verschlußelement (4) aus Silikon.

## Beschreibung

Die vorliegende Erfindung betrifft einen Single-Needle-Port, der subkutan implantierbar ist und zur Durchführung eine Hämodialyse oder Applikation einer Infusion dient. Derartige Single-Needle-Ports werden Patienten implantiert, die sich krankheitsbedingt mehrmals in der Woche beispielsweise einer Blutwäsche unterziehen müssen, etwa wegen einer Niereninsuffizienz. Dabei wird dem Patienten über einen zentralen Gefäßzugang oder über einen Shunt eine Punktionskanüle gesetzt. Das häufige Setzen der Kanüle führt zu einer starken Belastung des Hautgewebes in weiten Bereichen. Daher ist man seit längerem dazu übergegangen, derartigen Patienten einen sogenannten Port der vorliegenden Art zu implantieren, so daß lediglich eine Körperstelle bei der Vorbereitung der Hämodialyse wiederholt punktiert werden muß.

Rückseitig ist ein derartiger Single-Needle-Port dem Blutdruck des Patienten ausgesetzt. Dies bedeutet, daß dafür Sorge getragen werden muß, daß der Port, nachdem er durch die Punktionskanüle geöffnet worden ist und beispielsweise das Hämodialyseverfahren durchgeführt wurde, wieder sicher verschlossen wird, damit das unter dem eigenen Blutdruck stehende Blut des Patienten nicht aus dem Port austreten kann. Hier gibt es verschiedene Lösungsvorschläge, wie beispielsweise eine Membranlösung, bei der eine Punktionskanüle durch eine sich selbst wieder verschließende Membran in das Portinnere tritt. Dieser Vorschlag hat den Nachteil, daß die Membran aufgrund ihrer Beschaffenheit nur eine sehr begrenzte Anzahl der Öffnungen bzw. Schließungen des Ports zuläßt, bevor eine gewisse Leckage zu bemerken ist.

Vor diesem Hintergrund ist es die Aufgabe der vorliegenden Erfindung, hier Abhilfe zu schaffen, d.h. also einen Single-Needle-Port anzugeben, der seine Funktion über einen langen Zeitraum sicher ausübt, d.h. also sich sehr viel öfter als bekannte Ports öffnen und schließen läßt, wobei der Aufbau des Ports möglichst einfach und damit kostengünstig sein soll.

Gelöst wird diese Aufgabe durch einen Single-Needle-Port durch die Merkmale des Anspruchs 1. Vorteilhafte Weiterbildungen ergeben sich aus den Unteransprüchen.

Gemäß der Erfindung besteht der Single-Needle-Port aus einem im wesentlichen trichterförmigen Portgehäuse, dessen untere Trichteröffnung als Portöffnung ausgebildet ist und in dem das Verschlußelement angeordnet ist. Die trichterförmige Ausbildung des Portgehäuses dient der einfachen Implantation des Ports unter der Haut des Patienten. Das abdichtende Verschlußelement ist erfindungsgemäß pfropfenförmig ausgebildet und liegt mit seiner äußeren Mantelfläche in dichtender Anlage an dem Innenmantel des Portgehäuses an. Dabei besteht das Verschlußelement aus einem homogenen, elastischen Material, durch welches hindurch die Punktionskanüle bei Einleitung der Behandlung führbar ist, wobei das Material die Punktionskanüle in abdichtender Weise umschließt. Beim Herausziehen der Portkanüle aus dem Port entwickelt das Material solch hohe Rückstellkräfte, daß sich der von der Punktionskanüle zuvor ausgebildete Durchgangskanal wieder schließt.

Vorzugsweise besteht das Verschlußelement aus körperverträglichem Silikon. Dieses Material ist in hervorragender Weise geeignet, die Dichtfunktion über eine lange Zeit auszuüben. Die trichterförmige Ausbildung des Portgehäuses bietet neben der einfachen Implantierbarkeit des Ports die Sicherheit, daß das pfropfenförmige Verschlußelement beim Einführen der Punktionskanüle nicht weiter in das Gehäuse hineingedrängt werden kann.

Vorzugsweise wird das Verschlußelement an einem Herausziehen aus dem trichterförmigen Portgehäuse dadurch gehindert, daß der Rand des Portgehäuses an der oberen Trichteröffnung eingebördelt ist. Etwaige Tendenzen des Verschlußelementes, aufgrund des Reibschlusses zwischen der Punktionskanüle und dem Material des Verschlußelementes, aus dem Gehäuse herausgezogen zu werden, werden so wirksam unterbunden.

Gemäß einer vorteilhaften Weiterbildung ist an dem Portgehäuse wenigstens eine Lasche angeformt, durch welche wenigstens eine Durchbohrung verläuft. Diese wenigstens eine Durchbohrung dient beispielsweise dazu, daß das Portgehäuse mit der Haut vernäht werden kann, um etwaigen Migrationstendenzen des Portgehäuses entgegenzuwirken. Bei Implantation direkt auf einen Knochen können durch die Durchbohrung auch Knochenschrauben zur Fixation des Portgehäuses am Knochen geführt werden.

Dem gleichen Zweck dient eine weitere vorteilhafte Weiterbildung, bei der im Bereich der oberen Trichteröffnung des Portgehäuses eine Lasche angeformt ist, die wenigstens eine Durchbohrung aufweist. Auf diese Weise kann das Portgehäuse an mehreren Stellen vernäht oder verschraubt werden.

Besonders körperverträglich ist der Port, wenn das Portgehäuse aus Titan gefertigt ist.

Die Erfindung wird anhand eines Ausführungsbeispiels gemäß der Zeichnungsfiguren näher erläutert.

Hierbei zeigt:
- Figur 1: eine seitliche Schnittansicht durch den Single-Needle-Port,
- Figur 2: eine Ansicht des Ports in Richtung des Pfeiles A in Figur 1,
- Figur 3: eine Ansicht des Ports in Richtung des Pfeiles B in Figur 1, und
- Figur 4: eine Aufsicht auf das Portgehäuse.

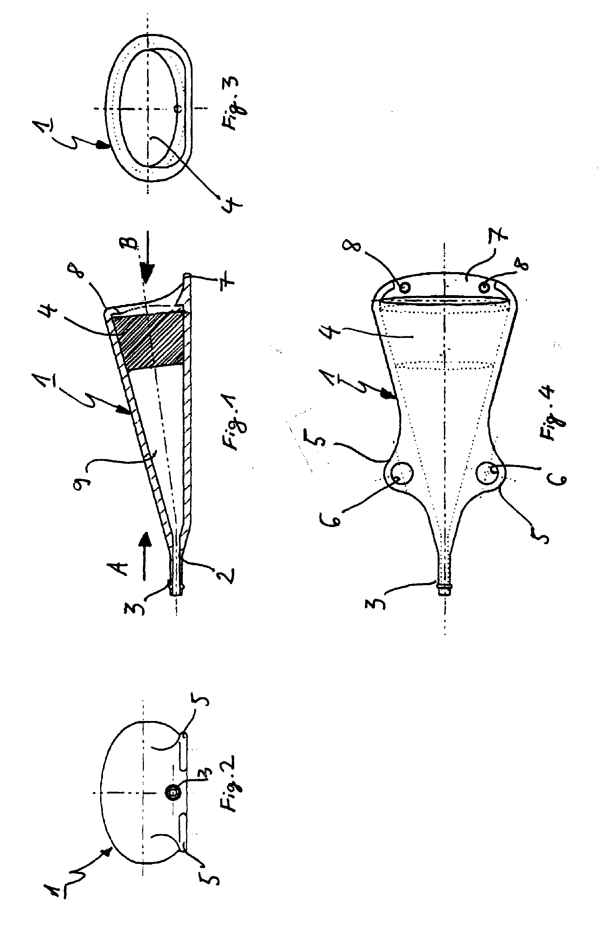

Den ersten Überblick verschafft Figur 1. Das darin dargestellte Portgehäuse 1 ist ersichtlich trichterförmig ausgebildet. Die untere Trichteröffnung 2 ist als Portöffnung 3 ausgebildet, d.h. als schnabelartiger Ansatz, über welchen ein Adapter führbar ist, welcher mit dem Blutgefäß in Verbindung gebracht wird. Die gegenüberliegende obere Trichteröffnung ist die Einführöffnung für eine Portkanüle (nicht dargestellt). Vorliegend ist der Rand 8 des Ports an der oberen Trichteröffnung eingebördelt. Die Einbördelung dient zum Zurückhalten des in dem Portgehäuse 1 untergebrachten Verschlußelementes 4. Das Verschlußelement 4 ist so ausgeformt und hat eine solche Größe, daß es mit seiner äußeren Mantelfläche in dichtender Anlage an dem Innenmantel des Portgehäuses anliegt. Diese Dichtung sorgt dafür, daß das im Hohlraum 9 des Ports unter dem eigenen Blutdruck stehende Blut nicht aus der oberen Trichteröffnung austreten kann.

Das Verschlußelement 4 besteht hier aus körperverträglichem Silikon. Das Portgehäuse 1 besteht aus Titan, welches besonders körperverträglich ist.

Zur Fixierung des Portgehäuses 1 im Patientenkörper entweder durch Vernähen mit der Haut oder durch Verschrauben an dem Knochen, sind vorliegend seitlich an das Portgehäuse 1 zwei Laschen 5 angeformt, welche jeweils durch eine Durchbohrung 6 durchsetzt ist. Durch diese Durchbohrung kann entweder ein Faden geführt werden oder eine Schraube gesetzt werden.

An der oberen Trichteröffnung ist darüber hinaus eine weitere Lasche 7 angeformt, welche mit zwei Durchbohrungen versehen ist. Auch diese zwei Durchbohrungen 8 dienen zur Fixierung des Portgehäuses 1.

## Patentansprüche

1. Single-Needle-Port, der subkutan implantierbar ist, bei dem die Portöffnung nach Beendigung der Behandlung mittels eines Verschlußelementes (4) abdichtbar ist und durch Einführung einer Punktionskanüle zur erneuten Behandlung zu öffnen ist, bestehend aus
einem im wesentlichen trichterförmigen Portgehäuse (1), dessen untere Trichteröffnung (2) als Portöffnung (3) ausgebildet ist und in dem das Verschlußelement (4) angeordnet ist, welches pfropfenförmig ausgebildet ist und mit seiner äußeren Mantelfläche in dichtender Anlage an dem Innenmantel des Portgehäuses (1) anliegt, wobei das Verschlußelement (4) aus einem homogenen, elastischen Material besteht, durch welches hindurch die Punktionskanüle führbar ist, wobei das Material die Punktionskanüle in abdichtender Weise umschließt, und welches beim Herausziehen der Portkanüle solch hohe Rückstellkräfte entwickelt, daß sich der von der Punktionskanüle ausgebildete Durchgangskanal wieder schließt.

2. Single-Needle-Port nach Anspruch 1, bei dem das Verschlußelement (4) aus Silikon besteht.

3. Single-Needle-Port nach Anspruch 1 oder 2, bei dem der Rand des Portgehäuses (1) an der oberen Trichteröffnung eingebördelt ist.

4. Single-Needle-Port nach einem der Ansprüche 1 bis 3, bei dem am Portgehäuse (1) wenigstens eine Lasche (5) angeformt ist, durch welche wenigstens eine Durchbohrung (6) verläuft.

5. Single-Needle-Port nach einem der Ansprüche 1 bis 4, bei dem das Portgehäuse (1) aus Titan besteht.

6. Single-Needle-Port nach einem der Ansprüche 1 bis 5, bei dem im Bereich der oberen Trichteröffnung des Portgehäuses (1) eine Lasche (7) angeformt ist, die wenigstens eine Durchbohrung (8) aufweist.
